# EUROPEAN PATENT SPECIFICATION

(11) **EP 2 184 286 B1**
(45) Date of publication and mention of the grant of the patent: **29.02.2012**
(21) Application number: 08019325.3
(22) Date of filing: 05.11.2008
(51) Int. Cl.: C07D 473/32

(54) **Process for the manufacture of acyclovir pro-drugs**
Verfahren zur Herstellung von Acyclovir-Prodrugs
Procédé de fabrication de pro-médicaments acyclovir

(43) Date of publication of application: 12.05.2010
(73) Proprietor: Cognis IP Management GmbH, 40589 Düsseldorf (DE); Universidad Complutense De Madrid, 28040 Madrid (ES)
(72) Inventor: Bigorra Llosas, Joaquin, 08201 Sabadell (ES); Valls, Ramon, 08022 Barcelona (ES); Raya, Javier, 08970 Sant Joan Despí (ES); Fabry, Bernd, 41352 Korschenbroich (DE); De Regil-Hernandez, Ruben, 28017 Madrid (ES); Rodriguez-Bayon, Amalia, 28034 Madrid (ES); Sinisterra, Jose-Vicent, 28013 Madrid (ES)

(56) References cited:
- US-A- 3 998 807
- GAO H ET AL: "NMR spectral data for acyclovir prodrugs" MAGNETIC RESONANCE IN CHEMISTRY, JOHN WILEY, CHICHESTER, GB, vol. 37, 1 January 1999 (1999-01-01), pages 687-689, XP002492474 ISSN: 0749-1581
- BANDO HIROTO ET AL: "Theoretical Design of Prodrug-Enhancer Combination Based on a Skin Diffusion Model: Prediction of Permeation of Acyclovir Prodrugs Treated with l-Geranylazacycloheptan-2-one" PHARMACEUTICAL RESEARCH, NEW YORK, NY, US, vol. 13, no. 3, 1 January 1996 (1996-01-01), pages 427-432, XP009112455 ISSN: 0724-8741

## Description

### Field of the invention

The present invention is related to the area of pharmaceuticals and concerns a new method for the manufacture of acyclovir pro-drugs, using green solvents.

### Background of the invention

Depending on the portal of entry Herpes virus are classified in *neurotrophic* (the infection in human body is through the nervous system), and *lymphotrophic* (the infection is through the lymphatic system). Herpes simplex virus (type 1 and 2: HSV-1 and HSV-2) and Varicella zoster (VZV) are among the neurotrophic virus while Human citomegalovirus (HCMV), Ep-stein-Barr virus (EBV) and Human herpes virus (HHV-6, HHV-7 and HHV-8) are among the lymphotrophic virus.

Herpes virus is a ubiquitous organism that causes infections in human population in the entire world. The overall incidence of herpes zoster in Europe is approximately 3 per 1000 people per year and more than 10 per 1000 per year in those aged > 80 years. The risk in a lifetime of being infected by herpes zoster is estimated at 10-20% and more than 50% in those aged > 80 years. Herpes simplex virus (HSV) infections are among the most common diseases in humans, with an estimated 60-95% of the adult population being infected by at least one of them. (HSV-1 is more frequently associated with oral and oro-labial diseases, whereas HSV-2 is more frequently associated with genital diseases.

Moreover, HSV is the most common cause of corneal blindness in the United States and it is also necessary to take into account the detrimental impact of pain on the patients' quality of life and the loss of productivity. The economic impact of Herpes zoster and post-herpetic neuralgia is largely underestimated in Europe. The high cost of healthcare in elderly patients is a serious concern in many countries. Since Herpes zoster and its complications are largely a disease of the elderly, the burden on healthcare resources will increase as the elderly population increases as shown by the census depicting a dramatic increase in the > 65 years age group up to the year 2025. Although hospitalization is a minor expense as for the total cost of the care for this disease, the annual cost of hospitalization for example in Spain was estimated at 7 M€.

Acyclovir, a synthetic 2'-desoxyguanosine analogue inhibits viral DNA replication without harming the host. Acyclovir is phosphorylated by the viral enzyme thymidine kinase, which is subsequently converted into triphosphate by cellular kinases in order to compete with normal desoxynucleoside triphosphate as a substrate for the viral DNA polymerase Acyclovir shows 30-50 times more affinity for viral DNA polymerase than for the host's. Also, in terms of toxicity, Acyclovir has demonstrated its safety for more than 30 years.

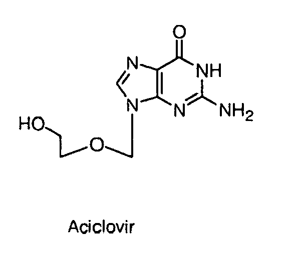

However, Acyclovir's success against HSV and VZV is limited by both its low oral bioavailability (15-20%) and low life time (t_{1/2} = 2.5-3 hours). These two factors have made necessary to increase Acyclovir oral dose and administration frequency as well, in order to achieve good clinical results. Moreover, Acyclovir low water solubility (≅0.2% at 25°C) is an inconvenient because it is impossible to use an Acyclovir formulation, for example, for intramuscular injection or as ophthalmic drops for ocular administration. In spite of the many research efforts, no good topic formulations of acyclovir have been obtained due to the impossibility of acyclovir to cross the skin corneal strata and reach the epidermis basal layer where the viral infection is present. To overcome this hindrance, different pro-drugs have been described without decisive results. As pro-drugs, one can cite: Valaciclovir, Penciclovir, Famciclovir, Ganciclovir and Valganciclovir. All these compounds are used by oral administration.

Therefore, topical application is a much more promising way of administrating Acyclovir to the patient. However, cosmetic or pharmaceutical formulations in general, as well as the employed solvents in particular have to fulfil some serious requirements:
- The compositions/solvents must show a sufficient solubility for Acyclovir;
- The compositions/solvents need to improve stability of Acyclovir;
- The solvents need to be toxicologically safe.

Typically, solvents like toluene, DMF or DMSO are used to produce Acyclovir prodrugs, since they show a high solubility for the product and accelerate the speed of the reaction. However, these solvents are toxic and therefore need to be removed completely (less than 5 ppm). This factor is of course connected with high technical efforts and expenses.

It is known from the state of the art to obtain Acyclovir prodrugs by acylation of Acyclovir with carboxylic acid anhydrides in the presence of dimethyl formamide [Magnetic Resonance in Chemistry Vol. 37(1), 687-689 (1999**)**]. Also acyl clorides are well known for this type of acylation [Pharm. Res. Vol. 13(3), 427-432 (1996**)**].

In addition, WO 07/147052 (Dr. Reddy's Lab. Ltd.) discloses a topical composition comprising acyclovir, a crystallisation inhibitor, a film-forming agent and a volatile solvent, like e.g. acetone. Although it is pointed out that the solvents evaporate when the composition is brought to skin, the formulations do not fulfil the safety obligations for pharmaceutical products. In addition, the solvents disclosed in the state of the art do not show an improvement in the stability of the active agent.

Therefore, the problem underlying the present invention is to modify existing/current processes for making Acyclovir pro-drugs by substituting conventional toxic solvents by nontoxic alternatives in order to avoid their removal from the composition. In addition, the nontoxic solvents used in this invention can be used in the topic formulation of acyclovir prodrugs.

### Detailed description of the invention

The present invention claims a process for making acyclovir pro-drugs by esterification of 9(2-hydroxyethyoxymethyl)-guanosine (Acyclovir) with an acylation agent in the presence of at least one dialkyl amide solvent, which is characterised in that said dialkyl amides are derived from carboxylic acids selected from the group consisting of capric acid, caprylic acid, caprinic acid, lauric acid, myrystic acid, palmitic acid, palmoleic acid, stearic acid, cetearic acid, oleic acid, elaidinic acid, linolic acid, linoleinic acid, conjugated linoleic acid, ricinoleic acid, 12-hydroxystearic acid, gadoleinic acid, arachidonic acid, erucic acid, behenic acid, lactic acid, glycolic acid or citric acid and their mixtures.

It has been observed that said specific dialkyl amides are useful solvents for the esterification in the manufacture of Acyclovir pro-drugs to achieve yields similar to those obtained with conventional solvents like toluene, DMF or DMSO. Toluene and DMF are toxic in dermo-pharmacy applications and DMSO is a crystalline opacificant. The major advantage of the alternative new solvents proposed is that they are toxicologically safe. Therefore a complete separation from the product is not necessary. Moreover, the pro-drug dissolved in the specific dialkyl amide could be directly incorporated into the final composition of the topical formulation. For this reason the economy of the production could strongly improved.

### Acyclovir pro-drugs

The chemical synthesis of acyclovir pro-drugs was described by Spector et al. [Biochem. Pharmacol. 32, p.2505-2509 (1983**)].** These scientists described the deamination of 2,6-diamino-9-(2-hydroxyethoximethyl) purine catalyzed by adenosine deaminase to give acyclovir. Then, Krenitsky et al. [Proc. Natl. Acad. Sci. 81, p.3209-3213 (1984**)]** synthesized 6-desoxiacyclovir which showed a good oral absorption. This compound was then transformed later into acyclovir by the xantine oxidase catalyzed oxidation. Nevertheless, both prodrugs showed higher toxicity than acyclovir. Kim et al, [Biotechnol. Bioeng. 57(1), p. 121-125 (1998**)]** synthesized 6-fluoropurine and 2-amino-6-fluoro-9-(2-hydroxiethoximethyl)purine have showed interesting properties as pro-drugs to be used by oral administration. Gao et al. [Synthesis 3, p. 329-351 (2000**)]** synthesized several alkanoates of acyclovir using fatty acid anhydrides ranging from acetic to palmitic anhydride acid. The synthesis took place in 2 or 3 days with the highest yields around 80%, but with low chemo-selectivity because the amide in C-6 was obtained (5-12%). Both problems disturb the scale up of the synthetic procedure. Finally, lipases have been used as selective biocatalysts to obtain nucleoside esters by Ferrero et al. [Chem. Rev. 100 (12), p. 4319-4347, (2000**)].**

### Acylation agents

The preparation of pro-drugs of Acyclovir requires an esterification step in which an acylation agent attacks the free terminal hydroxyl group of the acycloguanosine. The nature of the acylation is not critical for the invention, since the choice fully depends on the later application. Basically, said acylation agent can be
- a saturated or unsaturated, linear or branched monocarboxylic acid, having 1 to 22 carbon atoms, its chloride or anhydride, or
- a saturated or unsaturated dicarboxylic acid having 2 to 36 carbon atoms, its chloride or anhydride.

Typical examples are acetic acid, propionic acid, butyric acid, capric acid, caprylic acid, caprinic acid, lauric acid, myrystic acid, palmitic acid, palmoleic acid, stearic acid, cetearic acid, oleic acid, elaidinic acid, linolic acid, linoleinic acid, conjugated linoleic acid, ricinoleic acid, 12-hydroxystearic acid, gadoleinic acid, arachidonic acid, erucic acid, behenic acid and their mixtures. Also useful are short chain hydroxy carboxylic acids, like e.g. lactic acid, glycolic acid or citric acid. Suitable examples for dicarboxylic acids are malic acid, maleic acid, fumaric acid or adipic acid. Instead of the free acids it is useful to use their chlorides or preferably their anhydrides. The esterification takes place under conditions known to the ones skilled in the art. Usually, alkaline catalysts are added and the reaction conducted for about 12 to about 60, preferably about 24 to about 48 h at temperatures between about 15 to about 30 °C. Once the esterification is completed unreacted starting material is separated off either by distillation or - preferred - by washing with water.

### Dialkyl amides

The dialkyl amides that are useful as solvents according to the present invention are water soluble. The preferred amides are dimethylamides based on C₆-C₁₀ fatty acid, C₈-C₁₀ fatty acid, lactic acid and their mixtures. Typically, the dialkyl amides are used in concentrations of about 0.1 M to about 5.0 M, preferably 0.2 M to 2.0 M and more preferably 0.5 M to 1.0 M - calculated on the total reaction mixture.

### Industrial application

As described in the introduction acyclovir pro-drugs are efficiently used in dermopharmacy.

### Examples

### General Synthesis

### The synthesis of acyclovir pro-drugs is conducted according to the following reaction.

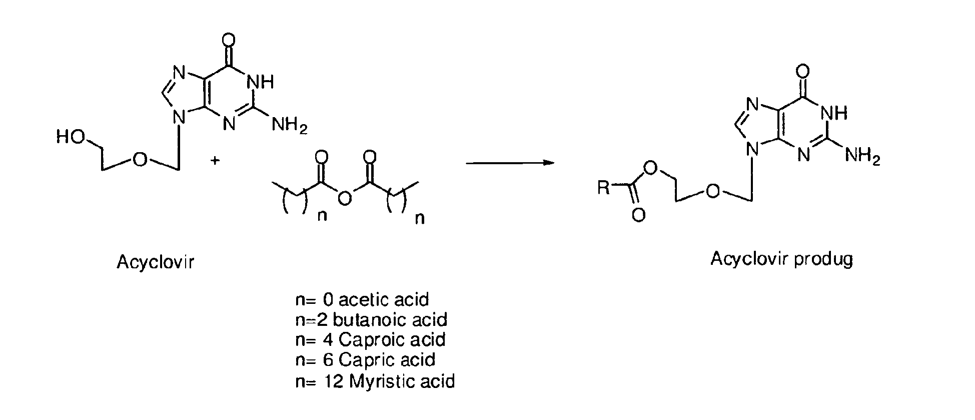

### Example 1

### Synthesis of acyclovir hexanoate using N,N-dimethylamide of decanoic acid

Under argon atmosphere, acyclovir (25 mM) and hexanoic anhydride (75 mM) were placed in 1 200 ml 3-necked flask and 3.5 ml of the solvent N,N-dimethyldecanoamide were added. Once 2 mM of the catalyst 4-N,N-dimethylaminopyridine were added, the mixture was stirred at 25°C for 48h. Subsequently the reaction mixture was washed with water to remove the unreacted acid and anhydride. The yield obtained as the isolated product was 90%. The ester was characterized by NMR, IR and TLC.

### Example 2

### Synthesis of the ester of acyclovir and octanoic acid

The reaction was performed as described in the Example 1 but using instead of hexanoic anhydride octanoic anhydride (60 mM). The yield obtained as isolated product was 73%.

### Example 3

### Synthesis of acyclovir hexanoate using N,N,-dimethylamide of octanoic acid.

The reaction was performed as described in Example 1 but using N,N-dimethylamide of octanoic acid as the solvent. The obtained yield as isolated ester was 60%

## Claims

1. A process for making acyclovir pro-drugs by esterification of 9(2-hydroxyethyoxymethyl)-guanosine (Acyclovir) with an acylation agent in the presence of at least one dialkyl amide solvent, **characterised in that** said dialkyl amides are derived from carboxylic acids selected from the group consisting of capric acid, caprylic acid, caprinic acid, lauric acid, myrystic acid, palmitic acid, palmoleic acid, stearic acid, cetearic acid, oleic acid, elaidinic acid, linolic acid, linoleinic acid, conjugated linoleic acid, ricinoleic acid, 12-hydroxystearic acid, gadoleinic acid, arachidonic acid, erucic acid, behenic acid, lactic acid, glycolic acid or citric acid and their mixtures.

2. A process according to Claim 1, **characterised in that** said acylation agent is a saturated or unsaturated, linear or branched monocarboxylic acid, having 1 to 22 carbon atoms, its chloride or anhydride.

3. A process according to Claim 1, **characterised in that** said acylation agent is a saturated or unsaturated dicarboxylic acid having 2 to 36 carbon atoms, its chloride or anhydride.

4. A process according to any of the preceding Claims 1 to 3, **characterised in that** said dialkyl amides are based on C₆-C₁₀ fatty acid, C₈-C₁₀ fatty acid, lactic acid or their mixtures.

## Patentansprüche

1. Verfahren zur Herstellung von Acyclovir-Prodrugs durch Veresterung von 9-(2-Hydroxyethoxymethyl)-guanosin (Acyclovir) mit einem Acylierungsmittel in Gegenwart mindestens eines Dialkylamid-Lösungsmittels, **dadurch gekennzeichnet, dass** sich die Dialkylamide von Carbonsäuren aus der Gruppe bestehend aus Capronsäure, Caprylsäure, Caprinsäure, Laurinsäure, Myristinsäure, Palmitinsäure, Palmitoleinsäure, Stearinsäure, Cetearinsäure, Ölsäure, Elaidinsäure, Linolsäure, Linolensäure, konjugierter Linolsäure, Ricinolsäure, 12-Hydroxystearinsäure, Gadoleinsäure, Arachidonsäure, Erucasäure, Behensäure, Milchsäure, Glykolsäure oder Citronensäure und deren Mischungen ableiten.

2. Verfahren nach Anspruch 1, **dadurch gekennzeichnet, dass** es sich bei dem Acylierungsmittel um eine gesättigte oder ungesättigte, lineare oder verzweigte Monocarbonsäure mit 1 bis 22 Kohlenstoffatomen, deren Chlorid oder Anhydrid handelt.

3. Verfahren nach Anspruch 1, **dadurch gekennzeichnet, dass** es sich bei dem Acylierungsmittel um eine gesättigte oder ungesättigte Dicarbonsäure mit 2 bis 36 Kohlenstoffatomen, deren Chlorid oder Anhydrid handelt.

4. Verfahren nach einem der vorhergehenden Ansprüche 1 bis 3, **dadurch gekennzeichnet, dass** die Dialkylamide auf C₆-C₁₀-Fettsäure, C₈-C₁₀-Fettsäure, Milchsäure oder deren Mischungen basieren.

## Revendications

1. Procédé de fabrication de promédicaments de l'acyclovir par estérification de la 9-(2-hydroxyéthoxy-méthyl)-guanosine (Acyclovir) à l'aide d'un agent d'acylation en présence d'au moins un solvant de type dialkylamide, **caractérisé en ce que** lesdits dialkylamides sont dérivés d'acides carboxyliques choisis dans le groupe constitué par l'acide caproïque, l'acide caprylique, l'acide caprinique, l'acide laurique, l'acide myristique, l'acide palmitique, l'acide palmitoléique, l'acide stéarique, l'acide cétéarique, l'acide oléique, l'acide élaïdinique, l'acide linolique, l'acide linolénique l'acide linoléique conjugué, l'acide ricinoléique, l'acide 12-hydroxystéarique, l'acide gadoléinique, l'acide arachidonique, l'acide érucique, l'acide béhénique, l'acide lactique, l'acide glycolique ou l'acide citrique et leurs mélanges.

2. Procédé conforme à la Revendication 1, **caractérisé en ce que** ledit agent d'acylation est un acide monocarboxylique linéaire ou ramifié, saturé ou insaturé, comportant entre 1 et 22 atomes de carbone, son chlorure ou son anhydride.

3. Procédé conforme à la Revendication 1, **caractérisé en ce que** ledit agent d'acylation est un acide dicarboxylique saturé ou insaturé, comportant entre 2 et 36 atomes de carbone, son chlorure ou son anhydride.

4. Procédé conforme à l'une quelconque des Revendications 1 à 3 précédentes, **caractérisé en ce que** lesdits dialkylamides sont basés sur un acide gras en C₆-C₁₀, un acide gras en C₈-C₁₀, l'acide lactique ou leurs mélanges.
